# EUROPEAN PATENT SPECIFICATION

(11) **EP 2 578 246 B1**
(45) Date of publication and mention of the grant of the patent: **01.06.2016**
(21) Application number: 12182347.0
(22) Date of filing: 30.08.2012
(51) Int. Cl.: A61L 27/22, A61L 27/50, A61L 27/38

(54) **Scaffold for vascular endothelial cell migration**
Gerüst für die vaskuläre endotheliale Zellmigration
Structure pour la migration de cellules endothéliales vasculaires

(30) Priority: 29.09.2011 JP 2011215549
(43) Date of publication of application: 10.04.2013
(73) Proprietor: Fujifilm Corporation, Minato-ku Tokyo (JP)
(72) Inventor: Iwazawa, Reiko, Kanagawa (JP); Nakamura, Kentaro, Kanagawa (JP)
(74) Representative: Hoffmann Eitle

(56) References cited:
- EP-A1- 2 428 215
- EP-A1- 2 543 397
- DATABASE WPI Week 200882 Thomson Scientific, London, GB; AN 2008-O19734 XP002733251, & WO 2008/133196 A1 (FUJI FILM CORP) 6 November 2008 (2008-11-06) -& WO 2008/133196 A1 (FUJIFILM CORP [JP]; OOYA SHOUJI [JP]; KOBAYASHI TAKAYUKI [JP]) 6 November 2008 (2008-11-06)
- DATABASE WPI Week 201102 Thomson Scientific, London, GB; AN 2010-Q59936 XP002733267, & WO 2010/147109 A1 (FUJI FILM CORP) 23 December 2010 (2010-12-23) -& WO 2010/147109 A1 (FUJIFILM CORP [JP]; OGIWARA KAZUTAKA [JP]; ISHIKAWA NORIKO [JP]; OOYA) 23 December 2010 (2010-12-23)

## Description

### BACKGROUND OF THE INVENTION

### Technical Field

The present invention relates to a scaffold for vascular endothelial cell migration.

### Description of the Related Art

Currently, an attempt to put the regenerative medicine into practical use, which aims to attain regeneration of biological tissues and organs suffering functional impairment or dysfunction, has been carried out. Regenerative medicine is a new medical technology which, in a living tissue that can no longer be restored solely by the natural healing ability of the living body, recreates the morphology and function similar to those of an intact tissue by utilizing three elements: cell, scaffold and growth factor.

It is well known that the most important factor that determines the success or failure of tissue generation referred to as the regenerative medicine is the success or failure of vascular introduction and induction of blood vessels toward the lesioned part. For tissue regeneration, delivery of nutrients is critical; therefore, it is considered important whether or not blood vessels, a means for delivering nutrients in the living body, are newly formed.

In order to induce angiogenesis, it has been proposed to administer a growth factor inducing angiogenesis (hereinafter, referred to as "angiogenic factor"), and examples of active ingredients thereof include vascular endothelial cell growth factors (VEGF), basic fibroblast growth factors (bFGF) and hepatocyte growth factors (HGF) (see, for example, J. Mol. Cell Cardiol. 33(3): 379-393 (2001)). These angiogenesis-inducing factors are humoral factors (growth factors) that exhibit an action to directly induce vascular endothelial cells.

Meanwhile, it is known that it is difficult to sufficiently induce and newly form desired blood vessels by using a humoral factor (growth factor) alone. In order to achieve more effective angiogenesis, scaffolds for induced vascular endothelial cells have been studied.

In the living body, a structurally stable substance, which is called extracellular matrix ("ECM") and surrounds the cells, is present, thereby providing a scaffold. Type-I collagen and fibronectin, both of which are known as major extracellular matrices, are involved in tissue repair, embryogenesis, blood coagulation and adhesion of vascular endothelial cells. These ECMs are used as a scaffold in angiogenesis as well (see, for example, Microvascular Research Volume 66, Issue 2, September 2003, pages 102-112).

In particular, fibronectin is recognized as a favorable ECM among currently available materials, and, therefore, a number of studies directed to the sequences that are effective for adhesion and migration of vascular endothelial cells have been conducted. From among these studies, for example, it is reported that RGD (arginine-glycine-aspartic acid) sequence and IGD (isoleucine-glycine-aspartic acid) sequence contribute to migration of vascular endothelial cells (see, for example, American Journal of Pathology vol.156, No.5 pp1673-1682, American journal of Pathology, vol.155, No.3, pp887-895 and Japanese National-Phase Patent Application Laid-open (JP-A) No. 2005-518409).

Further, Japanese Patent Application Laid-Open (JP-A) No. 2006-83095 discloses, as a compound applicable to vascular regeneration, a differentiation-promoting agent for stem cells and/or endothelial precursor cells, such as a specific pyrimidine compound, and describes that migration of vascular endothelial cells are promoted by exposing vascular endothelial cells to this compound.

WO2011108517 discloses crosslinked recombinant gelatin scaffolds for tissue regeneration.

### SUMMARY OF THE INVENTION

However, although the low-molecular-weight peptides and the low-molecular-weight compounds disclosed in the above-described documents promote migration, they do not serve as a scaffold for vascular endothelial cells.

Further, the evaluation of a material with respect to angiogenesis may employ evaluation criteria including ease of the adhesion of vascular endothelial cells, promotion of migration of vascular endothelial cells and ease of tube formation by vascular endothelial cells. From among these criteria, a scaffold material is strongly desired to, particularly, (1) allow good adhesion of vascular endothelial cells and (2) promotes migration of vascular endothelial cells, from the standpoint that the scaffold material is employed to guide blood vessels to a tissue. When the above-described ECMs are checked with respect to these criteria, it is known that although type-I collagen and fibronectin used as a scaffold material for angiogenesis have favorable properties with respect to the cell adhesion (1), they have insufficient properties with respect to cell migration ability (2). Further, even if individual peptide sequences which contribute to migration of vascular endothelial cells such as RGD or IGD are discovered, they are still insufficient for causing effective migration of vascular endothelial cells, and thus are difficult to use as an actual scaffold material. For the reasons discussed above, there is still a need for a scaffold material which exhibits high migration-promoting ability so as to ensure that blood vessels are guided to the tissue, .

An object of the present invention is to provide a scaffold for vascular endothelial cell migration which is capable of promoting migration of vascular endothelial cells more effectively as compared to conventional scaffolds.

The present invention is defined in the claims.

A scaffold for vascular endothelial cell migration which is capable of promoting migration of vascular endothelial cells more effectively as compared to conventional scaffolds can be provided.

### BRIEF DESCRIPTION OF THE DRAWINGS

Fig. 1 is a graph showing the cell migration rates in the respective scaffolds according to Example 1.
Fig. 2 is a graph showing the results of the cell migration evaluation according to Example 1.
Fig. 3 (A) is a stained image obtained when the scaffold containing CBE3 sponge and bFGF was implanted into a living body.
Fig. 3 (B) is an enlarged image of the boxed portion of Fig. 3 (A), according to Example 3 of the present invention.
Fig. 4 (A) is a stained image obtained when the scaffold containing only CBE3 sponge was implanted into a living body.
Fig. 4 (B) is an enlarged image of the boxed portion of Fig. 4 (A), according to Example 3 of the present invention.
Fig. 5 (A) is a stained image obtained when the scaffold containing an animal gelatin and bFGF was implanted into a living body.
Fig. 5 (B) is an enlarged image of the boxed portion of Fig. 5 (A), according to Example 3 of the present invention.

### DETAILED DESCRIPTION OF THE INVENTION

The scaffold for vascular endothelial cell migration according to the present invention contains a recombinant gelatin having an amino acid sequence derived from a partial amino acid sequence of collagen.

According to the present invention, more effective migration of vascular endothelial cells can be attained by using the scaffold for vascular endothelial cell migration that contains a recombinant gelatin having an amino acid sequence derived from a partial amino acid sequence of collagen, as compared to conventional scaffolds.

That is, the inventors of the present invention have found that the above-described recombinant gelatin contained in the scaffold for vascular endothelial cell migration according to the present invention markedly promotes migration of vascular endothelial cells. Therefore, use of the scaffold for vascular endothelial cell migration according to the present invention makes it possible to ensure that vascular endothelial cells migrate to a predetermined site to newly form blood vessels.

The term "step" used herein encompasses not only an independent step but also a step which cannot be clearly distinguished from other steps, as long as the expected effect of the step of the present invention is attained.

Further, numerical ranges described herein with "to" indicate a range which includes the numerical values stated before and after "to" as the minimum and maximum values, respectively.

In the present invention, in a case in which the composition contains plural substances corresponding to a particular component, the amount of the particular component in the composition described herein refers to the total amount of the plural substances present in the composition, unless otherwise specified.

The present invention is described in detail below.

The recombinant gelatin according to the present invention is a recombinant gelatin which has an amino acid sequence derived from a partial amino acid sequence of collagen.

Recombinant gelatins which have an amino acid sequence derived from a partial amino acid sequence of collagen can be used as the recombinant gelatin, and examples of recombinant gelatins that can be used include those disclosed in, for example, EP 1014176 A2, US 6992172, WO2004/85473, and WO2008/103041. The recombinant gelatin has a molecular weight of preferably from 2 kDa to 100 kDa, more preferably from 5 kDa to 90 kDa, and still more preferably from 10 kDa to 90 kDa.

The recombinant gelatin preferably has a repeating sequence represented by Gly-X-Y as the amino acid sequence derived from a partial amino acid sequence of collagen. This repeating sequence is a sequence characteristic to collagen. The plural Gly-X-Y may be the same or different. In the Gly-X-Y, Gly represents a glycine residue and X and Y each independently represent an given amino acid residue other than glycine. In the present invention, an amino acid residue in the gelatin is simply expressed as "amino acid", and a specific amino acid residue is simply expressed by the name of the amino acid corresponding to the specific amino acid residue, unless otherwise specified. Xs in the gelatin preferably include imino acid, specifically proline or oxyproline, at high proportion, and Ys in the gelatin preferably include imino acid, specifically proline or oxyproline, at high proportion. The content ratio of imino acid, such as those described above, is preferably from 10% to 45% of the whole recombinant gelatin. The content ratio (in terms of the number of amino acid residues) of the Gly-X-Y in the recombinant gelatin is preferably 80% or higher, more preferably 95% or higher, and most preferably 99% or higher, with respect to the whole recombinant gelatin.

From the standpoint of biocompatibility, the recombinant gelatin preferably contains a cell adhesion signal, and more preferably contains two or more cell adhesion signals in one molecule. Examples of the cell adhesion signal include RGD, LDV, REDV, YIGSR, PDSGR, RYVVLPR, LGTIPG, RNIAEIIKDI, IKVAV, LRE, DGEA and HAV sequences, and preferred examples include RGD, YIGSR, PDSGR, LGTIPG, IKVAV and HAV sequences. The cell adhesion signal is particularly preferably an RGD sequence. ERGD sequence is still more preferred among RGD sequences.

The arrangement of RGD sequences in the recombinant gelatin may be such that the number of amino acid residues between RGDs is preferably from 0 to 100, and more preferably from 25 to 60. The RGD sequences are preferably arranged unevenly with the number of amino acid residues therebetween within such a range.

In the recombinant gelatin, the ratio of the RGD sequences to the total number of amino acid residues (the ratio of the number of the amino acid residues belonging to the RGD sequences to the total number of amino acid residues) is preferably at least 0.4%, and in cases where the recombinant gelatin contains 350 or more amino acid residues, it is preferred that each stretch of 350 amino acid residues contain at least one RGD sequence.

The recombinant gelatin preferably contains at least two RGD sequences, more preferably at least three RGD sequences, and still more preferably at least four RGD sequences, per 250 amino acid residues. Further, the sequence of the recombinant gelatin preferably satisfies at least one of the following conditions (1) to (3): (1) include neither serine nor threonine; (2) does not include any of serine, threonine, asparagine, tyrosine and cysteine; and (3) does not include an amino acid sequence represented by Asp-Arg-Gly-Asp. The recombinant gelatin satisfy one of, or two or more of, the preferred conditions (1) to (3).

Further, the recombinant gelatin may be partially hydrolyzed.

The recombinant gelatin preferably has a repeating structure of A-[(Gly-X-Y)ₙ]ₘ-B. In the repeating structure, m represents 2 to 10, and preferably represents 3 to 5; A and B each independently represent an given amino acid or an amino acid sequence; and n represents 3 to 100, preferably 15 to 70, and more preferably 50 to 60.

Preferably, the recombinant gelatin is represented by the formula:

Gly-Ala-Pro-[(Gly-X-Y)₆₃]₃-Gly

In the formula, 63 Xs in each [(Gly-X-Y)₆₃] structure each independently represent an amino acid; 63 Ys in each [(Gly-X-Y)₆₃] structure each independently represent an amino acid; and the three [(Gly-X-Y)₆₃] structures may be the same as each other or different from each other.

The repeating units of the recombinant gelatin preferably includes plural repeats of the sequence unit of a naturally occurring collagen. Preferred examples of the "naturally occurring collagen" as mentioned herein include type-I, type-II, type-III, type-IV and type-V collagens. The "naturally occurring collagen" is more preferably type-I, type-II or type-III collagen. Preferred examples of the origin of the collagen include human, horse, pig, mouse and rat, and the origin of the collagen is more preferably human.

The isoelectric point of the recombinant gelatin is preferably from 5 to 10, more preferably from 6 to 10, and still more preferably from 7 to 9.5.

Examples of preferred properties that the recombinant gelatin may have include the followings: (1) not being deaminated; (2) not containing procollagen; (3) not containing telopeptide; and (4) being a substantially pure material for collagen which is prepared by nucleic acid encoding a natural collagen. The recombinant gelatin may have one of these preferred properties (1) to (4), or two or more thereof in combination.

From the standpoint of high migration-promoting ability, the recombinant gelatin is preferably any of the following (A) to (C).
(A) the polypeptide represented by the following SEQ ID NO:1:
   GAP(GAPGLQGAPGLQGMPGERGAAGLPGPKGERGDAGPKGADGAPGAPGLQGMP GERGAAGLPGPKGERGDAGPKGADGAPGKDGVRGLAGPIGPPGERGAAGLPGPKGE RGDAGPKGADGAPGKDGVRGLAGPIGPPGPAGAPGAPGLQGMPGERGAAGLPGPKG ERGDAGPKGADGAPGKDGVRGLAGPP)₃G (SEQ ID NO:1)
(B) a polypeptide having a homology of 80% or higher to the amino acid sequence of (A), and which has the ability to promote vascular endothelial cell migration; and
(C) a polypeptide having the same amino acid sequence as (A) except that one or a small number of amino acid residues are deleted, substituted or added, and which has the ability to promote vascular endothelial cell migration.

In (B), from the standpoint of the migration-promoting ability of the recombinant gelatin, the homology is more preferably 90% or higher, and still more preferably 95% or higher.

In (C), the number of amino acid residues deleted, substituted or added may be one or a small number, and varies depending on the total number of amino acid residues of the recombinant gelatin. The number of amino acid residues deleted, substituted or added is, for example, from 2 to 15, and preferably from 2 to 5.

The recombinant gelatin can be produced in accordance with a gene recombination technique known to those skilled in the art, such as the methods disclosed in EP 1014176 A2, US 6992172, WO2004/85473, WO2008/103041 and the like. Specifically, the recombinant gelatin may be produced as follows: a gene encoding the amino acid sequence of a predetermined recombinant gelatin is prepared, the gene is incorporated into an expression vector to prepare a recombinant expression vector, and the recombinant expression vector is introduced into an appropriate host to prepare a transformant. Since the recombinant gelatin is produced by culturing the obtained transformant in an appropriate medium, the recombinant gelatin for use in the present invention can be prepared by recovering the produced recombinant gelatin from the culture.

Here, the ability to promote vascular endothelial cell migration can be evaluated by a known cell migration test using a predetermined vascular endothelial cell line. Examples of the cell migration test applicable to the present invention include the migration test using a cell culture insert described below.

Preferable examples of recombinant gelatins applicable to the present invention include the recombinant gelatins disclosed in Japanese National-Phase Patent Application Laid-open (JP-A) Nos. 2010-519293, 2010-519252., 2010-518833, and No. 2010-519251, WO2010/128672, and WO2010/147109.

The scaffold for vascular endothelial cell migration (hereinafter sometimes simply referred to as "scaffold") contains the recombinant gelatin described above.

In order to allow vascular endothelial cells to migrate to the surface of the scaffold more efficiently, the amount of the recombinant gelatin in the surface of the scaffold is preferably not lower than 30 µg/cm², and more preferably not lower than 100 µg/cm². From the standpoint of efficient utilization of the recombinant gelatin, the amount of the recombinant gelatin in the surface of the scaffold may be not more than 3,000 µg/cm². When at least one of the above-described polypeptides (A) to (C) is employed as a preferred recombinant gelatin, the total amount of the polypeptides (A) to (C) in the surface of the scaffold is preferably not lower than 30 µg/cm², and more preferably not lower than 100 µg/cm², but is preferably not mope than 3,000 µg/cm², for the same reason as discussed above,.

The shape of the scaffold is not particularly restricted, and may be appropriately selected in accordance with, for example, the conditions and size of the region in which migration of vascular endothelial cells is desired as well as the site to which vascular endothelial cells are desired to migrate. For example, the scaffold may be sheet-shaped, rectangular or discoid. In order to obtain a scaffold of a particular shape, a support may also be used. The shape of the scaffold is not restricted to a particular shape. For example, a scaffold may be formed in a simple manner by applying the scaffold material, in a predetermined thickness, to a surface of a region in which vascular endothelial cells are desired to migrate.

From the standpoint of easily forming blood vessels having desired sizes, desired shapes, etc, the scaffold preferably has a rectangular or discoid shape with a predetermined thickness. The thickness of the scaffold is not particularly restricted, and may be, for example, from 0.1 mm to 50 cm, preferably from 0.5 mm to 10 cm, and more preferably from 1 mm to 1 cm.

The width and length of the scaffold may be set as appropriate, and are not particularly restricted. From the standpoint of, for example, forming a blood vessel having an appropriate size, the scaffold may have a diameter of 10 cm, and preferably has a diameter of 8 mm, in a case in which the scaffold is discoid.

From the standpoints of improving the migration of vascular endothelial cells and the angiogenesis by the vascular endothelial cells, the scaffold is preferably a three-dimensional porous body, and more preferably a three-dimensional sponge (spongy body). By using a three-dimensional porous body as the scaffold, migration of cells on the surface of the scaffold as well as migration of cells into the scaffold are more facilitated, so that more vascular endothelial cells are guided into the scaffold and the cell density inside the scaffold can be increased. Consequently, angiogenesis by the vascular endothelial cells that have migrated into the scaffold can be even more facilitated.

When the scaffold is a three-dimensional porous body, the scaffold may also contain other materials as long as its ability to promote migration of vascular endothelial cells attributable to the recombinant gelatin is not impaired. Nevertheless, it is preferred that the scaffold maintains its form by mainly containing the recombinant gelatin.

Here, the term "mainly" means that the ratio by mass of the component to the total mass of the three-dimensional porous body is the highest of all the components of the three-dimensional porous body. The three-dimensional porous body may be constituted by a single material or plural materials.

The mass ratio of the recombinant gelatin in the scaffold is preferably not lower than 90%, more preferably not lower than 95%, and most preferably 100%; that is, the shape of the scaffold can be maintained by the recombinant gelatin alone.

Here, the expression "the shape of the scaffold is maintained by the recombinant gelatin alone" allows the inclusion of other components that do not participate in the maintenance of the shape, such as a variety of humoral factors such as growth factors, in the scaffold, in addition to the recombinant gelatin that forms the scaffold.

The scaffold is a three-dimensional porous body, and the scaffold may also contain, in addition to the recombinant gelatin, other skeletal materials (supports) capable of contributing to the maintenance of the shape. Examples of the skeletal materials include silicone.

The scaffold may be cross-linked or not cross-linked. From the standpoint of the structural stability, the scaffold is preferably a cross-linked product of the recombinant gelatin. By using a cross-linked product of the recombinant gelatin, the shape of the three-dimensional porous body can be appropriately maintained by the recombinant gelatin alone, so that the ratio of the recombinant gelatin in the scaffold can be increased and the ability of the scaffold to promote migration of vascular endothelial cells can be further more improved.

The cross-linking method to be employed may be a known method such as thermal cross-linking, chemical cross-linking, cross-linking by an aldehyde (e.g. formaldehyde or glutaraldehyde), cross-linking by a condensing agent (e.g. carbodiimide or cyanamide), enzymatic cross-linking, photo-cross-linking, UV cross-linking, hydrophobic interaction, hydrogen bonding or ionic interaction.

Examples of chemical cross-linking agents include formaldehyde, glutaraldehyde, carbodiimide and cyanamide. Examples of photo-cross-linking include cross-linking by photo-irradiation of a macromolecule to which a photo-reactive group has been introduced; and cross-linking by photo-irradiation in the presence of a photosensitizer. Examples of the photo-reactive group include a cinnamyl group, a coumarin group, a dithiocarbamyl group, a xanthene dye and camphorquinone.

In a case in which cross-linking is performed using an enzyme, the enzyme is not particularly restricted as long as the enzyme works to cross-link polymeric material molecules. Preferred examples of the enzyme include transglutaminase and laccase, and the cross-linking can be most preferably performed using transglutaminase. Specific examples of proteins to be cross-linked using transglutaminase include the recombinant gelatin and other proteins having lysine and glutamine residues.

The transglutaminase may be of mammalian origin or of microorganism origin, and specific examples thereof include: ACTIVA Series manufactured by Ajinomoto Co., Inc.; transglutaminases of mammalian origin that are commercially available as reagents, such as guinea pig liver-derived transglutaminases, goat-derived transglutaminases and rabbit-derived transglutaminases manufactured by, for example, Oriental Yeast Co., Ltd., Upstate USA Inc., and Biodesign International; and human-derived blood coagulation factor (manufactured by Factor XIIIa; Haematologic Technologies, Inc.).

The cross-linking method is preferably a cross-linking method which utilizes a chemical cross-linking agent, or a thermal cross-linking method. The cross-linking method which utilizes a chemical cross-linking agent is more preferably cross-linking which uses glutaraldehyde as the chemical cross-linking agent.

An example of the method of cross-linking the recombinant gelatin is a two-step method including:
mixing a solution of the recombinant gelatin with a cross-linking agent; and
allowing the recombinant gelatin and the cross-linking agent to react with each other in the uniform solution obtained by the mixing.

The temperature at which the recombinant gelatin solution and cross-linking agent are mixed is not particularly restricted as long as the solution can be mixed. The temperature is preferably from 0°C to 100°C, more preferably from 0°C to 40°C, more preferably from 0°C to 30°C, more preferably from 3°C to 25°C, more preferably from 3°C to 15°C, more preferably from 3°C to 10°C, and particularly preferably from 3°C to 7°C.

The reaction of the recombinant gelatin solution and the cross-linking agent may be carried out at an elevated temperature. The reaction temperature in the uniform solution obtained by the mixing is not particularly restricted as long as cross-linking proceeds, and the reaction temperature is practically from -100°C to 200°C, preferably from 0°C to 60°C, more preferably from 0°C to 40°C, more preferably from 3°C to 25°C, more preferably from 3°C to 15°C, more preferably from 3°C to 10°C, and particularly preferably from 3°C to 7°C, in consideration of the denaturation and degradation of the polymeric material.

The polymeric material can be cross-linked even without using any cross-linking agent. The cross-linking method that does not use any cross-linking agent is not particularly restricted, and specific examples thereof include a thermal cross-linking method.

In a case in which the cross-linking method that does not use any cross-linking agent is performed, the reaction temperature is not particularly restricted as long as a cross-linking product is obtained. The reaction temperature is preferably from -100°C to 500°C, more preferably from 0°C to 300°C, more preferably from 50°C to 300°C, more preferably from 100°C to 250°C. and still more preferably from 120°C to 200°C.

The scaffold is a three-dimensional porous body, and the average pore size of the scaffold is not particularly restricted as long as the average pore size is large enough to allow cells to migrate into the scaffold. The average pore size is preferably from 10 µm to 400 µm, more preferably from 50 µm to 300 µm, and still more preferably from 70 µm to 200 µm.

The porosity of the scaffold is not particularly restricted as long as cells can exist inside the scaffold at a predetermined density, and the porosity of the scaffold is preferably from 81 % to 99.99%, and more preferably from 95.01 % to 99.9%. Here, the porosity of the scaffold can be determined from its bulk density (p) and true density (pc) using the following equation: porosity (P) = (1-ρ/ρc) × 100(%).

In the present invention, the term "three-dimensional porous body" means a scaffold having a three-dimensional structure having plural pores.

The method of forming the porous body is not particularly restricted, and any known method can be suitably employed. From the standpoint of enabling formation of a scaffold having a desired form in a simpler manner, it is preferable to use a predetermined amount of cross-linking agent. For example, in a case in which glutaraldehyde is used, the porosity of the porous body can be adjusted by changing the concentration of the recombinant gelatin. For example, the porosity of the porous body can be increased by lowering the concentration of the recombinant gelatin.

In addition to the recombinant gelatin, the scaffold may also contain a variety of humoral factors such as growth factors, preferably angiogenic factors. The selected humoral factor(s) is expected to exert a synergistic effect on the vascular endothelial cells of which migration is promoted by the recombinant gelatin.

Humoral factors that can be used in combination with the recombinant gelatin in the scaffold are not particularly restricted, and examples thereof include angiogenic factors, nerve growth factors (NGFs), adipokines and interferons. Thereamong, from the standpoints of promoting migration of vascular endothelial cells and angiogenesis, the humoral factor is preferably an angiogenic factor such as vascular endothelial cell growth factor (VEGF), basic fibroblast growth factor (bFGF), hepatocyte growth factor (HGF) or platelet-derived growth factor (PDGF), and among such angiogenic factors, a basic fibroblast growth factor (bFGF) is preferred.

For promoting migration of vascular endothelial cells and angiogenic effect, the scaffold contains the recombinant gelatin and basic fibroblast growth factor (bFGF), which is an angiogenic factor.

The type of bFGF is not particularly restricted, and may be naturally occurring bFGF or recombinant bFGF. The recombinant bFGF may be, for example, a commercially available product TRAFERMIN (fibroblast) manufactured by Kaken Pharmaceutical Co., Ltd.

In a case in which the scaffold contains bFGF, the content of bFGF in the scaffold is preferably from 0.01 to 10 µg/mm³, more preferably from 0.1 to 5 µg/mm³, and still more preferably from 0.3 to 1 µg/mm³, from the standpoint of attaining more efficient angiogenesis.

The three-dimensional porous body containing the recombinant gelatin and bFGF can preferably be used also as a scaffold for angiogenesis. Since the scaffold for angiogenesis contains the recombinant gelatin and bFGF and is in the form of a three-dimensional porous body, the scaffold has improved ability to promote migration of vascular endothelial cells, and ensures that angiogenesis occurs by vascular endothelial cells inside the scaffold.

The above-described details of the recombinant gelatin and the bFGF to be used in the scaffold for vascular endothelial cell migration, including the definitions and examples thereof, are directly applicable to the recombinant gelatin and the bFGF to be used in the scaffold for angiogenesis. The above-described details of the three-dimensional structure and the porous structure of the scaffold for vascular endothelial cell migration, including the definitions and examples thereof, are directly applicable to the scaffold for angiogenesis.

As described in the above, the scaffold for vascular endothelial cell migration and the scaffold for angiogenesis both exert a powerful effect in terms of promoting migration of vascular endothelial cells. Therefore, the scaffold for vascular endothelial cell migration and the scaffold for angiogenesis are effective for treatment of various diseases of which symptoms can be alleviated by migration of vascular endothelial cells. Examples of such diseases include ischemic diseases, diabetic skin ulcers, hearing impairments, cardiac diseases, acute coronary syndromes, acute myocardial infractions and unstable angina. Further, the scaffolds can preferably be used in various treatments such as cell and tissue regeneration therapies and cell transplantation treatments.

The scope of the present invention encompasses methods of treating such diseases. The method of treating a disease of which symptoms can be alleviated by migration of vascular endothelial cells includes disposing the scaffold for endothelial cell migration at a target site for migration of vascular endothelial cells.

The scope of the present invention also encompasses an angiogenesis method. The angiogenesis method includes disposing the scaffold for vascular cell migration or the scaffold for angiogenesis at a site at which angiogenesis is required.

In these treatment methods and angiogenesis method, the process for disposing the scaffold at a desired site is not particularly restricted, and known methods which do not adversely affect the treatment effect or the angiogenic effect of the scaffold for vascular endothelial cell migration and the scaffold for angiogenesis can be employed for the process.

The scaffold in the form of the three-dimensional porous body can preferably be applied as a scaffold for angiogenesis. Therefore, the scaffold can be employed in the production of blood vessels for regenerative medicine.

The scope of the present invention encompasses a method of preparing a blood vessel for regenerative medicine. The method of preparing a blood vessel for regenerative medicine includes bringing vascular endothelial cells isolated from a living body into contact with the scaffold and culturing the vascular endothelial cells in contact with the scaffold for vascular endothelial cell migration for a time required for angiogenesis.

Using this method, a blood vessel can be prepared *in vitro* from a material derived from a living body. The resulting blood vessel for regenerative medicine can be implanted into a subject in need thereof. In particular, in a case in which the vascular endothelial cells for use in the preparation of a blood vessel are collected from a subject in need of the blood vessel, and the blood vessel is prepared using the method described above, the possibility of rejection reaction is considerably low even when the resulting blood vessel is implanted into the subject (that is, autotransplanted). This is because the blood vessel is prepared from the vascular endothelial cells of the same subject. Therefore, the preparation of a blood vessel in this manner is particularly preferred.

In the method described above, the contact between the scaffold and the vascular endothelial cells, and the culturing of the vascular endothelial cells are preferably performed under sterile conditions as much as possible. Conventional culture conditions may be applied to the culturing.

The culturing period may be adjusted as appropriate in accordance with, for example, the size and length of the desired blood vessel or the number or condition of the vascular endothelial cells used.

The culture medium may be a medium which is usually used for culture of vascular endothelial cells, and examples thereof include Dulbecco's modified Eagle medium (DMEM) and RPMI 1640. A variety of additives applicable to an ordinary cell culture, such as serum, various vitamins and various antibiotic substances, may be added to the culture medium. From the standpoint of, for example, reducing the occurrence of rejection reaction after implantation, it is preferable to use a medium which does not contain any animal-derived component except a component obtained from the same species as the target subject for implantation.

A blood vessel for regenerative medicine obtained by the present method has a lumen. Therefore, when the blood vessel for regenerative medicine is applied to a living body as an implant, the blood vessel may serve to, for example, transport a body fluid such as blood therethrough. However, the blood vessel obtained by the present invention can be used in accordance with the conditions of the desired medical implant regardless of the presence or absence of a lumen, as long as the blood vessel performs a function as vascular wall.

### EXAMPLES

The present invention will now be described in detail by way of examples thereof. However, the present invention is not restricted to these examples. It is noted here that, unless otherwise specified, "%" is based on mass.

### [Example 1] (comparative)

### Evaluation of vascular endothelial cell migration-promoting ability in vitro

The ability of recombinant peptide CBE3 to promote the migration of human vascular endothelial cells was evaluated by the following evaluation tests. Migration is a process characteristic to angiogenesis and migration of cells facilitates angiogenesis. Two types of migration tests were performed: a test in which a base material was used as a scaffold and a test in which a base material was added to a culture medium.

### (1) Base material

The following CBE3 (described in WO2008/103041) was prepared as the recombinant gelatin according to the present invention.

CBE3:
Molecular weight: 51.6 kD
Structure: GAP[(GXY)₆₃]₃G
Number of amino acid residues: 571
Number of RGD sequences: 12
Imino acid content: 33%
The GXY repeating structure accounts for almost 100% of amino acid residues.

The amino acid sequence of this CBE3 does not contain any of serine, threonine, asparagine, tyrosine and cysteine.

CBE3 includes an ERGD sequence.

Isoelectric point: 9.34
Amino acid sequence (SEQ ID NO:1)

### (2) Migration Evaluation 1 - Sample Filter-

The substances tested were the CBE3, and bovine dermis-derived atelocollagen (Koken Co., Ltd.; acidic collagen solution I-PC, 5 mg/mL), human fibronectin (BD; fibronectin (human)) and porcine gelatin (Nippi, Inc.; high-grade gelatin APAT), to which the CBE3 was to be compared. The test substances were each dissolved with injection water (OTSUKA DISTILLED WATER; Otsuka Pharmaceutical Co., Ltd.).

A cell culture insert (BD Falcon; FLUOROBLOK separate-type cell culture insert, 24-well plate with 8-µm filter membrane) was used for the evaluation of cell migration. All operations were performed under sterile conditions. The insert was set on the plate, and the solution of each test substance was dripped onto the filter membrane. The resultant was then air-dried for 16 hours and evaluated.

Test substance solutions at concentrations of 5 µg/ml, 50 µg/ml, 500 µg/ml, 5,000 µg/ml and 50,000 µg/ml were used in the case of the CBE3 and the porcine gelatin, test substance solutions at concentrations of 5 µg/ml, 50 µg/ml, 500 µg/ml, and 5,000 µg/ml were used in the case of the collagen, and test substance solutions at concentrations of 5 µg/ml, 50 µg/ml and 500 µg/ml were used in the case of the fibronectin. Each of the solutions was dripped onto the filter membrane in an amount of 50 µL. The concentrations of 50,000 g/ml, 5,000 µg/ml, 500 µg/ml, 50 µg/ml and 5 µg/ml correspond to coating amounts of 8333 µg/cm², 833 µg/cm², 83.3 µg/cm², 8.33 µg/cm² and 0.83 µg/cm², respectively.

GFP-expressing human umbilical vein endothelial cells (AGP; GFP-HUVEC) were employed for the test. A 5 v/v% serum-containing medium EBM-2MV (Lonza) was used as a culture medium for proliferation, and a serum-free medium EBM-2 (Lonza) was used as a culture medium for the evaluation tests. The culture medium was added into the bottom of the wells of the 24-well plate in an amount of 800 µL, and the cell culture insert in which the cells were inoculated on the filter membrane was set on the plate. Here, the cells had been inoculated on the filter membrane in an amount of 200 µL at a concentration of 2.5 × 10⁵ cells/ml. The plate on which the cell culture insert was set was incubated in 5 v/v% CO₂ at 37°C for 6 hours.

The cell migration-promoting ability of test substances was evaluated by measuring the number of cells that moved from the upper side to the bottom side of the filter membrane through the filter membrane. By measuring the fluorescence intensity of each well using a fluorescence plate reader of bottom-excitation-and-bottom-measuring-type (ENVISION-2103 DISPENSER; Perkin-Elmer), the intensity of fluorescence from only the cells present on the bottom side of the filter membrane was determined. The results in terms of the ratio of the fluorescence intensity to the incubation time are shown in Table 1. From Table 1, it is seen that the highest migration-promoting effect was attained when the CBE3 was coated in an amount of 8,333 µg/cm². As for the collagen and the fibronectin, the highest migration-promoting effect was observed at a coating amount of 8.33 µg/cm².

**Table 1**

| Type of Base Material | Coating Amount (µg/cm²) | Fluorescence Intensity |
|---|---|---|
| CBE3 | 0.83 | 4247.80 |
| | 8.33 | 5419.73 |
| | 83.3 | 7354.16 |
| | 833 | 10114.03 |
| | 8333 | 17886.76 |
| Collagen | 0.83 | 1535.77 |
| | 8.33 | 12214.11 |
| | 83.3 | 10902.30 |
| | 833 | 2239.32 |
| | 8333 | - |
| Fibronectin | 0.83 | 1877.86 |
| | 8.33 | 9224.89 |
| | 83.3 | 5612.26 |
| | 833 | - |
| | 8333 | - |
| Porcine gelatin | 0.83 | 1781.74 |
| | 8.33 | 3436.87 |
| | 83.3 | 3106.71 |
| | 833 | 4006.42 |
| | 8333 | 1832.09 |

In order to quantify the residual amount of the base material coated on the filter, the amount of amino acids was quantified. Specifically, o-phthalaldehyde was allowed to react with the amino acids, the fluorescent intensity was measured, and the amount of amino acids was calculated.

A filter membrane on which the base material was coated by the same method as described above was used for the preparation of a calibration curve. Further, the same filter membrane was mock-cultured in PBS (pH 7.2) (Gibco) at 37°C for 6 hours, and the resulting filter membrane was used for quantification. Here, the mock culture was performed by adding 800 µL of PBS to the wells of 24-well plate, setting the cell culture insert thereon, adding 200 µL of PBS to the upper side of the insert, and incubating the resulting plate at 37°C in 5% CO₂.

The degradation of the base material on the filter membrane into amino acids was carried out by the following method.

To the same cell culture insert plate as that used in the migration ability evaluation test, 376 µL of 0.4 M boric acid (Reagent Special Grade; Wako Pure Chemical Industries, Ltd.) buffer (pH 10) and 376 µL of 1 N aqueous sodium hydroxide solution (for Volumetric Analysis; Wako Pure Chemical Industries, Ltd.) were added. Then, the cell culture insert was set on the plate. The wells were sealed with a sterilized aluminum plate seal (manufactured by As One Corporation), the plate was covered with a lid, and reaction was allowed to proceed for 16 hours at a temperature of 80°C and a humidity of 90%. After the reaction, the plate was cooled to room temperature, and the seal was removed. Then, the reaction solution on the cell culture insert was transferred to the wells, and the cell culture insert was removed. To the wells to which the reaction solution was transferred, 441 µL of 0.4 M boric acid buffer (pH 10) was further added.

The quantification of amino acids was carried out using o-phthalaldehyde (OPA) (Wako Pure Chemical Industries, Ltd.) and N-acetylcysteine (NAC) (Wako Pure Chemical Industries, Ltd.). The OPA was dissolved in methanol (manufactured by Wako Pure Chemical Industries, Ltd.) to form a 160 mg/ml solution, and the OPA solution was added to NAC dissolved in the above-described boric acid buffer, such that the OPA solution corresponded to 1 v/v% of the resultant NAC solution. The resultant NAC solution having a concentration of 2 mg/ml NAC (also containing of OPA) in an amount of 294 µL was added to the wells, and the solutions in the wells were allowed to react at 37°C for 15 minutes while the solutions were agitated using a plate shaker. Immediately thereafter, the fluorescence intensity was measured using a plate reader (ENVISION-2103 DISPENSER; Perkin-Elmer).

The results are shown in Table 2. The collagen and the fibronectin in amounts similar to the original coating amounts remained on the filter membrane even after 6 hours of culturing. In contrast, the amounts of the CBE3 and the porcine gelatin that remained on the filter membrane after the culture were much lower than the original coating amounts. In the case of the CBE3, the residual ratio on the filter membrane was only 2% when the coated amount was 8,333 µg/cm², only 3.6% when the coating amount was 833 µg/cm², and only 10% when the coating amount was 83.3 µg/cm². It is noted here that, for the same base material, the residual amount varies in accordance with the coating amount, and the dissolution rate of the base material into the medium was rather uniform regardless of the coating amount.

**Table 2**

| Type of Base Material | Coating Amount (µg/cm²) | Residual Amount (µm/cm²) |
|---|---|---|
| CBE3 | 0.83 | 1.69 |
| | 8.33 | 3.52 |
| | 83.3 | 8.71 |
| | 833 | 30.39 |
| | 8333 | 160.81 |
| Collagen | 0.83 | 5.23 |
| | 8.33 | 7.42 |
| | 83.3 | 110.12 |
| 833 | 833 | 644.34 |
| | 8333 | - |
| Fibronectin | 0.83 | 10.09 |
| | 8.33 | 19.49 |
| | 83.3 | 96.10 |
| | 833 | - |
| | 8333 | - |
| Porcine gelatin | 0.83 | 1.01 |
| | 8.33 | 4.14 |
| | 83.3 | 13.06 |
| | 833 | 25.23 |
| | 8333 | 92.80 |

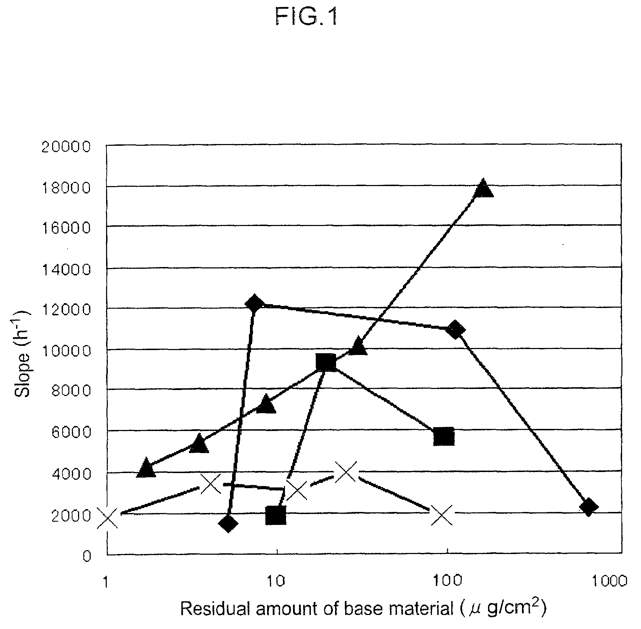

The relationships between the amount of the base material remaining on the filter and the migration-promoting ability (slope: the ratio of the fluorescence intensity to the incubation time) as determined by the fluorescence intensity are shown in Fig. 1. It is noted here that solid triangle, solid rhombus, solid square and × represent the CBE3, the collagen, the fibronectin and the porcine gelatin, respectively.

As shown in Fig. 1, the highest promotion rates in the samples were as follows:
the CBE3: 17,887/h
the collagen: 12,214/h
the fibronectin: 9,224/h.

When the CBE3 was coated in an amount of 8,333 µg/cm², the promotion rate was 1.5 times that observed with a collagen coating amount of 8.33 µg/cm², and 1.9 times that observed with a fibronectin coating amount of 8.33 µg/cm². It can be confirmed that, although the migration-promoting ability of the CBE3 increased depending on the residual amount thereof, the migration-promoting ability of the collagen, fibronectin and porcine gelatin does not depend on the residual amount. Since the migration-promoting ability of the other base materials than the CBE3 does not increase even when the residual amounts thereof were increased, it can be seen that the CBE3 has the highest migration-promoting ability.

### (2) Migration evaluation 2 - sample solution -

The substances tested were the CBE3, and bovine dermis-derived atelocollagen (Koken Co., Ltd.; acidic collagen solution I-PC, 5 mg/mL), human fibronectin (BD; fibronectin (human)) and porcine gelatin (Nippi, Inc.; high-grade gelatin APAT), to which the CBE3 was to be compared. In order to examine the migration-promoting effect of RGD sequence, a cyclic RGD (hereinafter, referred to as "cRGD") (CYCLO-RGFfK; AnaSpec, Inc.) was also used as a test substance. The test substances were each dissolved with injection water (OTSUKA DISTILLED WATER; Otsuka Pharmaceutical Co., Ltd.).

A cell culture insert (BD Falcon; FLUOROBLOK separate-type cell culture insert, 24-well plate with 8µm filter membrane) was used for the evaluation of cell migration. All operations were performed under sterile conditions.

GFP-expressing human umbilical vein endothelial cells (AGP; GFP-HUVEC) were employed for the test. A 5 v/v% serum-containing medium EBM-2MV (Lonza) was used as a culture medium for proliferation, and a serum-free medium EBM-2 (Lonza) was used as a culture medium for the evaluation tests. Test substance solutions at concentrations of 5 µg/ml, 50 µg/ml, 500 µg/ml, 5,000 µg/ml and 50,000 µg/ml were mixed in the case of the CBE3 and the porcine gelatin, test substance solutions at concentrations of 5 µg/ml, 50 µg/ml, 500 µg/ml, and 5,000 µg/ml were mixed in the case of the collagen, test substance solutions at concentrations of 5 µg/ml, 50 µg/ml and 500 µg/ml were mixed in the case of the fibronectin, and test solutions at concentrations of 0.7 µg/ml, 7 µg/ml, 70 µg/ml, 700 µg/ml and 7,000 µg/ml were mixed in the case of the cRGD. Each of the solutions of the respective test substances, in a volume of one tenth of the volume of the resultant mixture, was mixed with the serum-free medium EBM-2. The series of concentrations for the cRGD as described above were selected, so that the number of RGD sequences contained in the each cRGD solution was the same as the number of RGD sequences contained in an equivalent amount of its corresponding CBE3 solution in the concentration series. For example, the CBE3 concentrations of 5 µg/ml and 50,000 µg/ml correspond to the cRGD concentrations of 0.7 µg/ml and 7,000 µg/ml, respectively, in terms of the RGD sequence concentration.

In the other samples than the cRGD, the concentrations of 50,000 µg/ml, 5,000 µg/ml, 500 µg/ml, 50 µg/ml and 5 µg/ml in the test sample solutions correspond to contents of 5,000 µg, 500 µg, 50 µg, 5 µg and 0.5 µg, respectively, in one well. Also, the cRGD concentrations of 7,000 µg/ml, 700 µg/ml, 70 µg/ml, 7 µg/ml and 0.7 µg/ml in the cRGD solutions result in cRGD contents of 700 µg, 70 µg, 7 µg, 0.7 µg and 0.07 µg, respectively, in one well.

Each of the obtained mixture solutions, in an amount of 800 µL, was added into the bottom of wells of the 24-well plate, and the cell culture insert in which the cells were inoculated on the filter membrane was set on the plate. Here, the cells had been suspended in the respective base material mixed solutions and inoculated on the filter membrane in an amount of 200 µL at a concentration of 2.5 × 10⁵ cells/ml. The plate on which the cell culture insert was set was incubated at 37°C in 5 v/v% CO₂ for 6 hours.

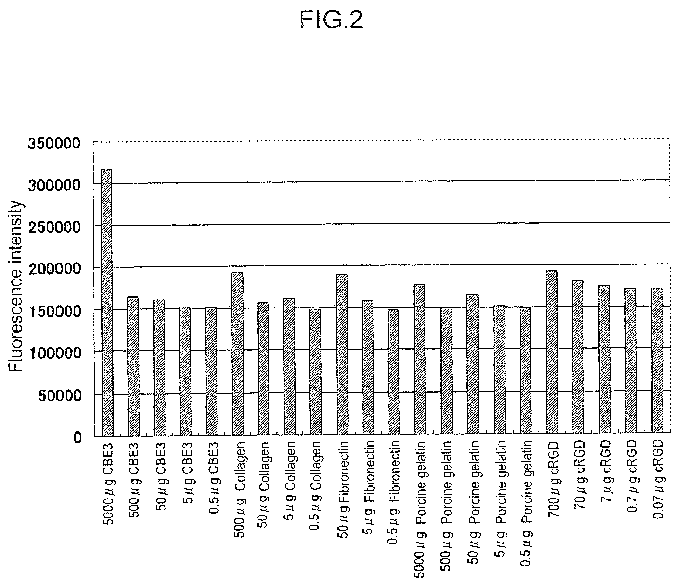

The cell migration-promoting ability of test substances was evaluated by measuring the number of cells that moved from the upper side to the bottom side of the filter membrane through the filter membrane. By measuring the fluorescence intensity of each well using a fluorescence plate reader of bottom-excitation-and-bottom-measuring-type (ENVISION-2103 DISPENSER; Perkin-Elmer), the intensity of fluorescence from only the cells present on the bottom side of the membrane was determined. The results are shown in Figure 2.

When the CBE3 was used in an amount of 5,000 µg, the highest migration -promoting effect was exhibited. In the case of the collagen, high migration promoting effect was observed at a content of 500 µg. In the case of the fibronectin, highest migration promoting effect was observed at a concentration of 50 µg. When the content of the CBE3 was 5,000 µg, the migration promoting effect was 1.6 times that observed when the collagen content was 500 µg, and 1.7 times that observed when the fibronectin content was 50 µg. When 700 µg of the cRGD, which contained the same amount of RGD sequences as that contained in 5,000 µg of the CBE3 molecules, was used, the migration-promoting effect was not as high as that exhibited by 5,000 µg of the CBE3. Thus, it can also be seen that the migration-promoting effect is not dependent on the cRGD content. From this, it was shown that the migration-promoting effect of the CBE3 is attributable to a factor which cannot be explained by the effects provided by the RGD sequence.

By the results of the above-described migration evaluations (1) and (2), it was found that migration of GFP-HUVEC cells is best promoted by using the CBE3. Further, it was also found that the promotion of migration of GFP-HUVEC cells by the CBE3 is dependent on the concentration of the CBE3.

### [Example 2]

### In vivo evaluation of angiogenesis-promoting ability

In order to investigate whether or not CBE3 can be a scaffold for angiogenesis *in vivo*, the following test was performed. A sponge that contains CBE3 (hereinafter, sometimes referred to as "CBE3 sponge") was subcutaneously implanted to a mouse, and angiogenesis in the CBE3 sponge was evaluated.

The CBE3 sponge was prepared by the following method.

A sample solution having the following composition was prepared, and glutaraldehyde was added thereto. Thereafter, the resultant was stirred at 4°C at 17,000 rpm for 4 minutes using a homogenizer (AM-11; manufactured by Nippon Seiki Co., Ltd.), and rapidly cooled at -80°C for 3 hours.

### Sample solution

Composition: 10 mL of 5% CBE3 liquid for sponge forming (CBE3: 500 mg; ultrapure water: 9,424 µL; 1N HCl: 76 µL; 3% glutaraldehyde: 500 µL)

Then, the resulting sample was left to stand at 4°C for 16 hours, followed by shaking for 4 hours in a sufficient amount of 0.2 M glycine solution at 37°C. Thereafter, the resultant was washed with 10 L of ultrapure water 8 times (for a total of 4 hours), frozen at -80°C for 2 hours, and then freeze-dried for 4 hours using a freeze-dryer, as a result of which a CBE3 sponge was obtained.

The average pore size of the obtained CBE3 sponge as determined by observation of its internal cross-sectional structure under a scanning electron microscope was 100 µm.

The porosity was determined from the bulk density (p) and true density (pc) using the following equation: porosity (P) = (1-ρ/ρc) × 100(%). Based on the bulk density (p) calculated from the dry weight and the volume, and the true density (pc) determined by a method using a Hubbard-type pycnometer, the porosity (P) was found to be not less than 95%.

An animal gelatin sponge (manufactured by Astellas Pharma Inc.; SPONGEL) was used as a comparative example for comparison with the CBE3 sponge.

The sponges were shaped in the following manner. Each sponge was cut out into the form of a disk having a diameter of 8 mm and a thickness of 3 mm. The obtained discoid sponges were each placed in a tube (SERUMTUBE, Sumitomo Bakelite Co., Ltd.), which was then sealed in a bag (ELK BAG; Cosmo Bio Co., Ltd.), and the bag was subjected to ethylene oxide sterilization (EOG sterilization) using an EOGELK SA-N160 (Elk Corporation).

The sponges to be implanted were infiltrated with bFGF (FIBROBLAST SPRAY; Kaken Pharmaceutical Co.,Ltd.) or physiological saline (Otsuka Pharmaceutical Co., Ltd.). A bFGF solution having a concentration of 1 mg/ml in PBS (pH 7.2; GIBCO) was prepared. The sponge was placed on a 24-well plate (non-treatment type; BD), and 80 µL of the bFGF liquid or physiological saline was dripped thereon. The resulting plate was covered with a lid and wrapped in a plastic wrap so as to prevent evaporation, and was then incubated at 4°C for 18 hours.

5-week-old male ddY mice (SLC) were employed as mice to be implanted with the sponge.

Implantation was performed by the following procedures. First, under isoflurane inhalation anesthesia, an incision of about 2 cm was made on the back of the neck of each mouse using scissors. From this incision, scissors were inserted and the skin adhering to the muscle was detached all the way to a position about 2 cm to 3 cm from the tail. The incubated sponge was pinched with forceps and inserted from the incision made on the neck by sliding down the forceps along the skin, thereby placing the sponge at a position about 2 cm to 3 cm from the tail. The incision made on the neck was then closed using a surgical stapler (Natsume Seisakusho Co., Ltd.), and wiped with cloth impregnated with ISODINE (Meiji Seika Kaisha, Ltd.). After the implantation, the mice were reared for 2 weeks.

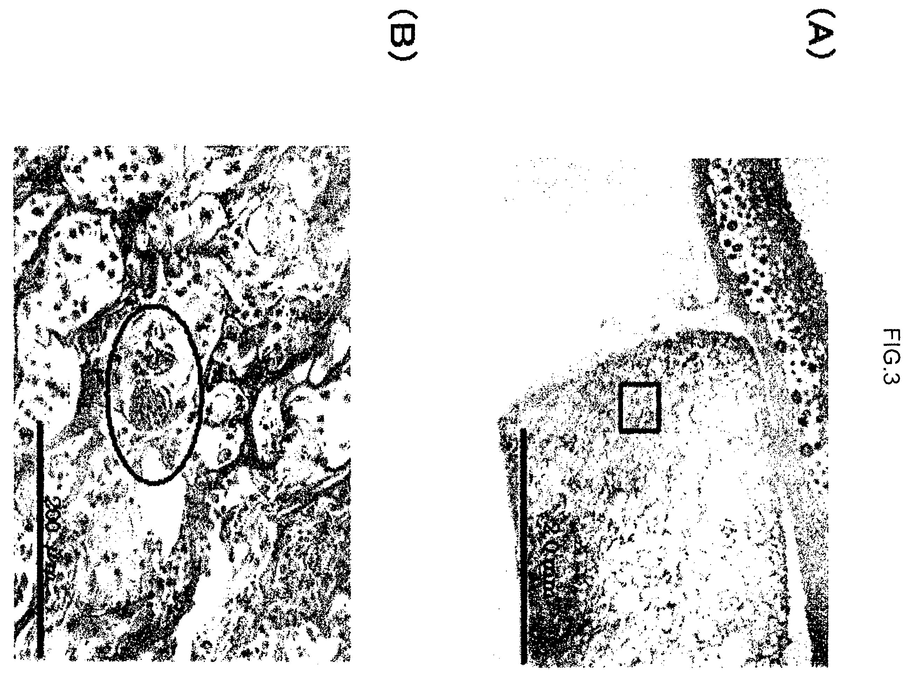

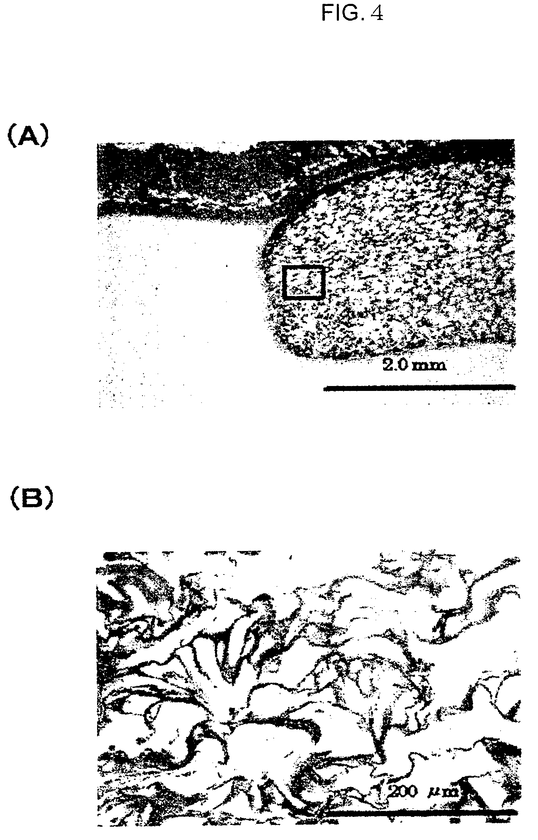

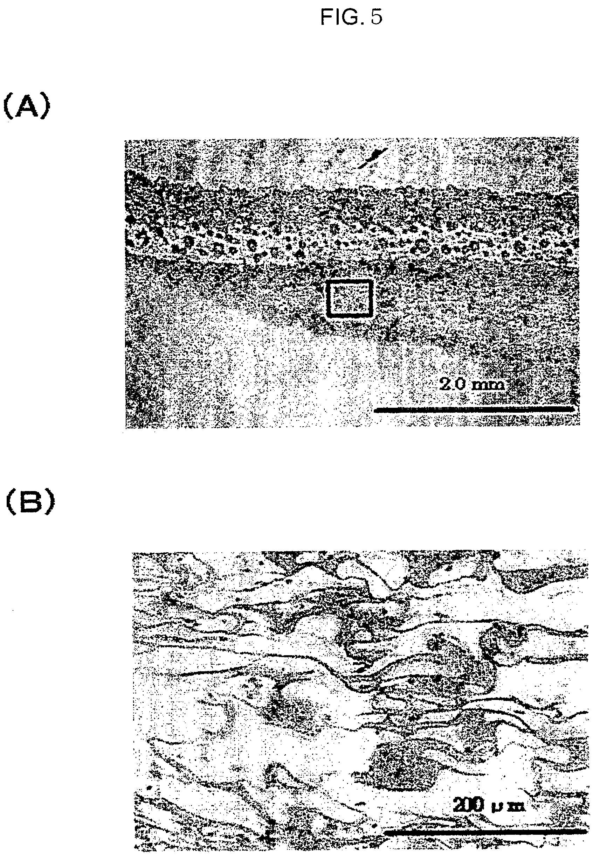

The sponge to be evaluated was taken out of each mice 2 weeks after the implantation. Autopsy was performed by the following method. First, using scissors, the skin was peeled off from below the neck wound to the tail. Then, a 1.5 cm by 1.5 cm square piece was cut out of the portion of the skin to which the sponge adhered by using a scalpel, and immersed in 4% paraformaldehyde (Wako Pure Chemical Industries, Ltd.). A cross-section at the center of the sponge was prepared by cutting the square piece in the transverse direction with respect to the back of the mouse, and a paraffin section was prepared therefrom. The paraffin section was subjected to HE staining. The results are shown in Figs. 3A to 5B. In the figures, the bar in Figs 3A, 4A and 5A represents 2.0 mm, and the bar in Figs. 3B, 4B and 5B represents 200 µm. In each of Figs. 3A, 4A and 5A, the skin appears at the upper left area, and the sponge appears in the lower right area. In the CBE3 sponge in with the bFGF in Fig. 3B, red blood cells are observed in the lumen, and vascular wall cells are observed around the lumen. This confirms the formation of blood vessel (see the circled portion of Fig. 3B). In contrast, in the CBE3 sponge without the bFGF (see Fig. 4B) and the animal gelatin sponge with the bFGF (see Fig. 5B), no angiogenesis was observed.

Evaluations were conducted by the following method.

First, an upper part, a middle part and a lower part were randomly selected from the area at the sponge side of the HE-stained section, and the images thereof were taken under a microscope (OLYMPUS IX71IX81). Then, the number of blood vessels and the area thereof in the images were quantified. The number of blood vessels was determined by counting blood vessels in each image, and the area of blood vessels was calculated using an IMAGEJ (NIH). The results thereof are shown in Table 3. The number of blood vessels is indicated in parentheses.

In each of the sponges, the same results were obtained for the 3 spots with regard to the presence or absence of blood vessels. Angiogenesis was confirmed in the CBE3 sponge with the bFGF. No angiogenesis was observed in the animal gelatin sponge with the bFGF and the CBE3 sponge without the bFGF. In the case of the CBE3 sponge without the bFGF , 0 blood vessels were formed on average, and the area of blood vessels was 0 µm² on average. In the case of the animal gelatin sponge with the bFGF, 0 blood vessels were formed on average, and the area of blood vessels was 0 µm² on average. In the case of the CBE3 sponge with the bFGF, 4.2 blood vessels were formed on average, and the area of blood vessels was 2,079 µm² on average. Accordingly, it can be seen that significant blood vessel formation occurred in the CBE3 sponge in the presence of the bFGF.

**Table 3**

| | | Blood vessel area [µm²] (Number of blood vessels) | | | |
|---|---|---|---|---|---|
| | | Upper part | Middle part | Lower part | Average |
| | Mouse 1 | 2726 (7) | 4857 (4) | 1617 (2) | 2079 (4.2) |
| CBE3 + bFGF | Mouse 2 | 0 (0) | 2970 (9) | 1211 (6) | |
| | Mouse 3 | 1609 (5) | 3722 (7) | 0 (0) | |
| | Mouse 4 | 0 (0) | 0 (0) | 0 (0) | 0 (0) |
| CBE3 | Mouse 5 | 0 (0) | 0 (0) | 0 (0) | |
| | Mouse 6 | 0 (0) | 0 (0) | 0 (0) | |
| SPONGEL + bFGF | Mouse 7 | 0 (0) | 0 (0) | 0 (0) | 0 (0) |
| | Mouse 8 | 0 (0) | 0 (0) | 0 (0) | |

As shown above, a scaffold containing the recombinant gelatin according to the present invention is capable of promoting migration of vascular endothelial cells. Further, when the scaffold is a three-dimensional porous body containing bFGF, angiogenesis is promoted inside the scaffold.

Therefore, a scaffold for vascular endothelial cell migration which is more effective in terms of allowing migration of vascular endothelial cells as compared to conventional scaffolds can be provided.

## Claims

1. A method for producing a blood vessel for regenerative medicine comprising:
bringing vascular endothelial cells isolated from a living body into contact with a scaffold for vascular endothelial cell migration comprising a recombinant gelatin having an amino acid sequence derived from a partial amino acid sequence of collagen, and
culturing the vascular endothelial cells in contact with the scaffold for a period required for angiogenesis
wherein the scaffold is in the form of a three-dimensional porous body, and comprises a basic fibroblast growth factor.

2. The method for producing a blood vessel for regenerative medicine according to claim 1, wherein the recombinant gelatin is a cross-linked recombinant gelatin.

3. The method for producing a blood vessel for regenerative medicine according to claim 1 or claim 2, wherein the recombinant gelatin comprises a cell adhesion signal and repeating sequence unit represented by Gly-X-Y, wherein X and Y each independently represent a given amino acid residue.

4. The method for producing a blood vessel for regenerative medicine according to any one of claims 1 to 3, wherein the recombinant gelatin comprises a cell adhesion signal and a repeating sequence unit represented by A-[(Gly-X-Y)n]m-B, wherein X and Y each independently represent a given amino acid residue, m represents an integer of from 2 to 10, A and B each independently represent a given amino acid residue or an amino acid sequence, and n represents an integer of from 3 to 100.

5. The method for producing a blood vessel for regenerative medicine according to any one of claims 1 to 4, wherein the recombinant gelatin is at least one selected from the group consisting of the following (A), (B) and (C):
(A) a polypeptide represented by SEQ ID 1;
(B) a polypeptide having homology of 80% or higher to the amino acid sequence of (A) and having an ability to promote vascular endothelial cell migration; and
(C) a polypeptide having the same amino acid sequence as (A) except that one or a small number of amino acid residues are deleted, substituted or added, and having an ability to promote vascular endothelial cell migration.

6. The method for producing a blood vessel for regenerative medicine according to any one of claims 1 to 5, wherein a content ratio of the recombinant gelatin to a total mass of the scaffold is highest among components of the scaffold.

7. The method for producing a blood vessel for regenerative medicine according to any one of claims 1 to 6, wherein the regenerative medicine is for ischemic diseases, diabetic skin ulcers, hearing impairments, cardiac diseases, acute coronary syndromes, acute myocardial infractions, unstable angina, promoting angiogenesis, cell and tissue regeneration therapies, and cell transplantation treatments.

## Patentansprüche

1. Verfahren zur Herstellung eines Blutgefäßes für die regenerative Medizin, umfassend:
in Kontakt bringen von vaskulären Endothelzellen, die aus einem lebenden Körper isoliert worden sind, mit einem Gerüst für die Migration von vaskulären Endothelzellen, umfassend eine rekombinante Gelatine, die eine Aminosäuresequenz aufweist, die aus einer Teil-Aminosäuresequenz von Kollagen abgeleitet ist, und
Kultivieren der vaskulären Endothelzellen in Kontakt mit dem Gerüst für eine Zeitspanne, die für die Angiogenese erforderlich ist,
worin das Gerüst in der Form eines dreidimensionalen porösen Körpers ist und einen basischen Fibroblast-Wachstumsfaktor umfasst.

2. Verfahren zur Herstellung eines Blutgefäßes für die regenerative Medizin gemäß Anspruch 1, worin die rekombinante Gelatine eine vernetzte rekombinante Gelatine ist.

3. Verfahren zur Herstellung eines Blutgefäßes für die regenerative Medizin gemäß Anspruch 1 oder Anspruch 2, worin die rekombinante Gelatine ein Zelladhäsionssignal und eine Wiederholungssequenzeinheit, die durch Gly-X-Y dargestellt wird, worin X und Y jeweils unabhängig einen gegebenen Aminosäurerest darstellen, umfasst.

4. Verfahren zur Herstellung eines Blutgefäßes für die regenerative Medizin gemäß irgendeinem der Ansprüche 1 bis 3, worin die rekombinante Gelatine ein Zelladhäsionssignal und eine Wiederholungssequenzeinheit, die durch A-[(Gly-X-Y)n]m-B. dargestellt wird, umfasst, worin X und Y jeweils unabhängig einen gegebenen Aminosäurerest darstellen, m eine ganze Zahl von 2 bis 10 darstellt, A und B jeweils unabhängig einen gegebenen Aminosäurerest oder eine Aminosäuresequenz darstellen, und n eine ganze Zahl von 3 bis 100 darstellt.

5. Verfahren zur Herstellung eines Blutgefäßes für die regenerative Medizin gemäß irgendeinem der Ansprüche 1 bis 4, worin die rekombinante Gelatine zumindest eine ist, die ausgewählt aus der Gruppe, bestehend aus den folgenden (A), (B) und (C):
(A) ein durch SEQ ID 1 dargestelltes Polypeptid;
(B) ein Polypeptid mit einer Homologie von 80 % oder höher zu der Aminosäuresequenz von (A) und mit der Fähigkeit, die Migration von vasuklären Endothelzellen zu fördern; und
(C) ein Polypeptid mit der gleichen Aminosäuresequenz wie (A), außer dass einer oder eine kleine Anzahl von Aminosäureresten entfernt, ersetzt oder zugegeben sind, und mit der Fähigkeit, die Migration von vaskulären Endothelzellen zu fördern.

6. Verfahren zur Herstellung eines Blutgefäßes für die regenerative Medizin gemäß irgendeinem der Ansprüche 1 bis 5, worin der Mengenanteil der rekombinanten Gelatine zur Gesamtmasse des Gerüsts unter allen Komponenten des Gerüsts am höchsten ist.

7. Verfahren zur Herstellung eines Blutgefäßes für die regenerative Medizin gemäß irgendeinem der Ansprüche 1 bis 6, worin die regenerative Medizin für ischämische Krankheiten, diabetische Hautgeschwüre, Hörbeeinträchtigungen, Herzkrankheiten, akute Koronarsyndrome, akuter Herzinfarkt, instabile Angina, zur Förderung der Angiogenese, für Zell- und Gewebe-Regenerationstherapien und für Zell-Transplantationsbehandlungen ist.

## Revendications

1. Procédé de production d'un vaisseau sanguin à des fins de médecine régénérative, comprenant :
la mise en contact de cellules endothéliales vasculaires prélevées sur un corps vivant avec une structure de migration de cellules endothéliales vasculaires comprenant une gélatine recombinante possédant une séquence d'acides aminés dérivée d'une séquence partielle d'acides aminés de collagène, et
la culture des cellules endothéliales vasculaires en contact avec la structure pendant une période nécessaire pour l'angiogenèse,
dans lequel la structure se présente sous la forme d'un corps poreux tridimensionnel et comprend un facteur basique de croissance des fibroblastes.

2. Procédé de production d'un vaisseau sanguin à des fins de médecine régénérative selon la revendication 1, dans lequel la gélatine recombinante est une gélatine recombinante réticulée.

3. Procédé de production d'un vaisseau sanguin à des fins de médecine régénérative selon la revendication 1 ou la revendication 2, dans lequel la gélatine recombinante comprend un signal d'adhérence cellulaire et un motif répétitif de séquence représenté par Gly-X-Y, où X et Y représentent chacun indépendamment un résidu d'acide aminé donné.

4. Procédé de production d'un vaisseau sanguin à des fins de médecine régénérative selon l'une quelconque des revendications 1 à 3, dans lequel la gélatine recombinante comprend un signal d'adhérence cellulaire et un motif répétitif de séquence représenté par A-[(Gly-X-Y)n]m-B, où X et Y représentent chacun indépendamment un résidu d'acide aminé donné, m représente un nombre entier de 2 à 10, A et B représentent chacun indépendamment un résidu d'acide aminé donné ou une séquence d'acides aminés et n représente un nombre entier de 3 à 100.

5. Procédé de production d'un vaisseau sanguin à des fins de médecine régénérative selon l'une quelconque des revendications 1 à 4, dans lequel la gélatine recombinante est au moins une gélatine choisie dans le groupe constitué des suivantes (A), (B) et (C) :
(A) un polypeptide représenté par la SEQ ID 1 ;
(B) un polypeptide présentant une homologie supérieure ou égale à 80 % avec la séquence d'acides aminés de (A) et étant capable de favoriser la migration de cellules endothéliales vasculaires ; et
(C) un polypeptide possédant la même séquence d'acides aminés que celle de (A), sauf qu'un résidu d'acide aminé ou un petit nombre de résidus d'acides aminés est supprimé, substitué ou ajouté, et étant capable de favoriser la migration de cellules endothéliales vasculaires.

6. Procédé de production d'un vaisseau sanguin à des fins de médecine régénérative selon l'une quelconque des revendications 1 à 5, dans lequel le rapport en teneur de la gélatine recombinante à la masse totale de la structure est le plus élevé d'entre les composants de la structure.

7. Procédé de production d'un vaisseau sanguin à des fins de médecine régénérative selon l'une quelconque des revendications 1 à 6, dans lequel la médecine régénérative est destinée aux maladies ischémiques, aux ulcères cutanés diabétiques, aux troubles de l'audition, aux maladies cardiaques, aux syndromes coronariens aigus, aux infarctus aigus du myocarde, aux angines instables, à la promotion de l'angiogenèse, aux thérapies par régénération cellulaire et tissulaire et aux traitements par transplantation de cellules.


### SEQUENCE LISTING

<110> FUJIFILM CORPORATION
<120> Scaffold for migration of vascular endothelial cells
<130> 11F03443
<150> JP2011-215549
   <151> 2011-09-29
<160> 11
<170> PatentIn version 3.4
<210> 1
   <211> 571
   <212> PRT
   <213> Artificial
<220>
   <223> CBE3
<400> 1
   Gly Ala Pro Gly Ala Pro Gly Leu Gln Gly Ala Pro Gly Leu Gln Gly
   1               5                   10                  15
   Met Pro Gly Glu Arg Gly Ala Ala Gly Leu Pro Gly Pro Lys Gly Glu
               20                  25                  30
   Arg Gly Asp Ala Gly Pro Lys Gly Ala Asp Gly Ala Pro Gly Ala Pro
           35                  40                  45
   Gly Leu Gln Gly Met Pro Gly Glu Arg Gly Ala Ala Gly Leu Pro Gly
       50                  55                  60
   Pro Lys Gly Glu Arg Gly Asp Ala Gly Pro Lys Gly Ala Asp Gly Ala
   65                  70                  75                  80
   Pro Gly Lys Asp Gly Val Arg Gly Leu Ala Gly Pro Ile Gly Pro Pro
                   85                  90                  95
   Gly Glu Arg Gly Ala Ala Gly Leu Pro Gly Pro Lys Gly Glu Arg Gly
               100                 105                 110
   Asp Ala Gly Pro Lys Gly Ala Asp Gly Ala Pro Gly Lys Asp Gly Val
           115                 120                 125
   Arg Gly Leu Ala Gly Pro Ile Gly Pro Pro Gly Pro Ala Gly Ala Pro
       130                 135                 140
   Gly Ala Pro Gly Leu Gln Gly Met Pro Gly Glu Arg Gly Ala Ala Gly
   145                 150                 155                 160
   Leu Pro Gly Pro Lys Gly Glu Arg Gly Asp Ala Gly Pro Lys Gly Ala
                   165                 170                 175
   Asp Gly Ala Pro Gly Lys Asp Gly Val Arg Gly Leu Ala Gly Pro Pro
               180                 185                 190
   Gly Ala Pro Gly Leu Gln Gly Ala Pro Gly Leu Gln Gly Met Pro Gly
           195                 200                 205
   Glu Arg Gly Ala Ala Gly Leu Pro Gly Pro Lys Gly Glu Arg Gly Asp
       210                 215                 220
   Ala Gly Pro Lys Gly Ala Asp Gly Ala Pro Gly Ala Pro Gly Leu Gln
   225                 230                 235                 240
   Gly Met Pro Gly Glu Arg Gly Ala Ala Gly Leu Pro Gly Pro Lys Gly
                   245                 250                 255
   Glu Arg Gly Asp Ala Gly Pro Lys Gly Ala Asp Gly Ala Pro Gly Lys
               260                 265                 270
   Asp Gly Val Arg Gly Leu Ala Gly Pro Ile Gly Pro Pro Gly Glu Arg
           275                 280                 285
   Gly Ala Ala Gly Leu Pro Gly Pro Lys Gly Glu Arg Gly Asp Ala Gly
       290                 295                 300
   Pro Lys Gly Ala Asp Gly Ala Pro Gly Lys Asp Gly Val Arg Gly Leu
   305                 310                 315                 320
   Ala Gly Pro Ile Gly Pro Pro Gly Pro Ala Gly Ala Pro Gly Ala Pro
                   325                 330                 335
   Gly Leu Gln Gly Met Pro Gly Glu Arg Gly Ala Ala Gly Leu Pro Gly
               340                 345                 350
   Pro Lys Gly Glu Arg Gly Asp Ala Gly Pro Lys Gly Ala Asp Gly Ala
           355                 360                 365
   Pro Gly Lys Asp Gly Val Arg Gly Leu Ala Gly Pro Pro Gly Ala Pro
       370                 375                 380
   Gly Leu Gln Gly Ala Pro Gly Leu Gln Gly Met Pro Gly Glu Arg Gly
   385                 390                 395                 400
   Ala Ala Gly Leu Pro Gly Pro Lys Gly Glu Arg Gly Asp Ala Gly Pro
                   405                 410                 415
   Lys Gly Ala Asp Gly Ala Pro Gly Ala Pro Gly Leu Gln Gly Met Pro
   420 425 430
   Gly Glu Arg Gly Ala Ala Gly Leu Pro Gly Pro Lys Gly Glu Arg Gly
   435 440 445
   Asp Ala Gly Pro Lys Gly Ala Asp Gly Ala Pro Gly Lys Asp Gly Val
   450 455 460
   Arg Gly Leu Ala Gly Pro Ile Gly Pro Pro Gly Glu Arg Gly Ala Ala
   465 470 475 480
   Gly Leu Pro Gly Pro Lys Gly Glu Arg Gly Asp Ala Gly Pro Lys Gly
   485 490 495
   Ala Asp Gly Ala Pro Gly Lys Asp Gly Val Arg Gly Leu Ala Gly Pro
   500 505 510
   Ile Gly Pro Pro Gly Pro Ala Gly Ala Pro Gly Ala Pro Gly Leu Gln
   515 520 525
   Gly Met Pro Gly Glu Arg Gly Ala Ala Gly Leu Pro Gly Pro Lys Gly
   530 535 540
   Glu Arg Gly Asp Ala Gly Pro Lys Gly Ala Asp Gly Ala Pro Gly Lys
   545 550 555 560
   Asp Gly Val Arg Gly Leu Ala Gly Pro Pro Gly
   565 570
<210> 2
   <211> 7
   <212> PRT
   <213> Artificial
<220>
   <223> recombinant gelatin
<220>
   <221> REPEAT
   <222> (4) .. (6)
   <223> [(Gly Xaa Xaa)x63]x3
<220>
   <221> MISC_FEATURE
   <222> (5) .. (6)
   <223> Xaa is any amino acid
<400> 2
   Gly Ala Pro Gly Xaa Xaa Gly
   1 5
<210> 3
   <211> 4
   <212> PRT
   <213> Artificial
<220>
   <223> cell adhesion signal sequence
<400> 3
   Arg Glu Asp Val
   1
<210> 4
   <211> 5
   <212> PRT
   <213> Artificial
<220>
   <223> cell adhesion signal sequence
<400> 4
   Tyr Ile Gly Ser Arg
   1               5
<210> 5
   <211> 5
   <212> PRT
   <213> Artificial
<220>
   <223> cell adhesion signal sequence
<400> 5
   Pro Asp Ser Gly Arg
   1               5
<210> 6
   <211> 7
   <212> PRT
   <213> Artificial
<220>
   <223> cell adhesion signal sequence
<400> 6
   Arg Tyr Val Val Leu Pro Arg
   1               5
<210> 7
   <211> 6
   <212> PRT
   <213> Artificial
<220>
   <223> cell adhesion signal sequence
<400> 7
   Leu Gly Thr Ile Pro Gly
   1 5
<210> 8
   <211> 10
   <212> PRT
   <213> Artificial
<220>
   <223> cell adhesion signal sequence
<400> 8
   Arg Asn Ile Ala Glu Ile Ile Lys Asp Ile
   1 5 10
<210> 9
   <211> 5
   <212> PRT
   <213> Artificial
<220>
   <223> cell adhesion signal sequence
<400> 9
   Ile Lys Val Ala Val
   1 5
<210> 10
   <211> 4
   <212> PRT
   <213> Artificial
<220>
   <223> cell adhesion signal sequence
<400> 10
   Asp Gly Glu Ala
   1
<210> 11
   <211> 4
   <212> PRT
   <213> Artificial
<220>
   <223> cell adhesion signal sequence
<400> 11
   Glu Arg Gly Asp
   1